# EUROPEAN PATENT SPECIFICATION

(11) **EP 1 267 642 B1**
(45) Date of publication and mention of the grant of the patent: **05.03.2008**
(21) Application number: 01919008.1
(22) Date of filing: 06.04.2001
(51) Int. Cl.: A23L 1/308, A23L 1/0522, A23L 1/30, A23L 1/29, A61K 31/718, A61K 31/20, A61P 3/04, A61P 3/06, A61P 3/08, A61P 3/10

(54) **COMPOSITION COMPRISING RESISTANT STARCH AND UNSATURATED FATS**
ZUSAMMMENSETZUNG ENTHALTEND RESISTENTE STÄRKE UND UNGESÄTTIGTE FETTE
COMPOSITION COMPRENANT DE L'AMIDON RÉSISTANT ET DES LIPIDES INSATURÉES

(30) Priority: 06.04.2000 AU PQ673300
(43) Date of publication of application: 02.01.2003
(73) Proprietor: Penford Australia Limited, Lane Cove, NSW 2066 (AU)
(72) Inventor: BROWN, Ian, Lewis, Gymea, NSW 2227 (AU); STORLIEN, Leonard, Henry, S-431 39 Molndal (SE); BROWN, Marc, Andrew, Tualatin, OR 97062 (US); HIGGINS, Janine, Denver, CO 80262 (US); TAPSELL, Linda, Clare, Stanwell Park, NSW 2058 (AU)
(74) Representative: Rees, Kerry
(86) International application number: PCT/AU2001/000392
(87) International publication number: WO 2001/076394

(56) References cited:
- EP-A- 0 506 166
- EP-A- 0 550 060
- EP-A- 0 747 397
- EP-A- 0 846 704
- WO-A-00/38537
- WO-A-97/35889
- DE-A1- 19 503 993
- US-A- 5 776 887
- ASP N.G. ADVANCES IN EXPERIMENTAL MEDICINE AND BIOLOGY vol. 427, 1997, pages 201 - 210, XP002961417
- KRIS-ETHERTON P.M.: 'Is there an optimal diet for the hypertriglyceridemic patient?' JOURNAL OF CARDIOVASCULAR RISK vol. 7, no. 5, October 2000, pages 333 - 337, XP002961418
- NOAKES M. ET AL AMERICAN JOURNAL OF CLINICAL NUTRITION vol. 64, no. 6, 1996, pages 944 - 951
- LIU G.C. ET AL METABOLISM vol. 32, 1983, pages 750 - 753

## Description

### Field of the invention

The present invention relates to the regulation of fat or lipid metabolism by means of compositions and diets which are high in resistant starch and unsaturated fats.

### Background to the invention

Obesity and overweight in general as well as a range of metabolic diseases such as non-insulin dependent diabetes mellitus, dislipidemias, hypertension and coronary heart disease are a widespread problem in large parts of the world. In many cases, the underlying cause of these diseases is the development of insulin resistance. The factors which contribute to the onset and development of insulin resistance have not been fully elucidated but the type of fat and/or carbohydrate in the diet have been exposed as crucial factors (Storlien et al., 1993, Diabetes, 42: 457-462).

Dietary starch, an important component of human carbohydrate intake, is composed of two types of glucose polymer, namely amylose and amylopectin. Amylose is a linear polymer of glucose residues linked by α 1-4) bonds whereas amylopectin is a branched polymer of glucose residues linked by α (1-4) and α(1-6) bonds.

Ingestion of amylopectin starch is known to produce a rapid and prolonged rise in plasma insulin and glucose concentrations which has been postulated to be detrimental to whole body insulin sensitivity in the long term. In humans, consumption of foods which cause a large rise in postprandial plasma glucose concentration is also associated with an increased concentration of free fatty acids in the plasma. This increase in plasma free fatty acid concentration causes a decrease in glucose oxidation. presumably via the glucose-fatty acid cycle, which may impair insulin sensitivity.

Asp, N.G. (1997). Advances in Experimental Medicine, vol. 427, p. 201-210 discloses resistant starch as having various physiological effects such as on colon fermentation, reduction in glycemic index, improvement in mineral absorption and reduction in plasma cholesterol.

EP-A-550 060 discloses a composition comprising an enzyme resistant starch which reduces blood cholesterol. and obesity risk.

US-A-5 776 887 discloses a nutritional composition for the dietary management of diabetics comprising slowly absorbed carbohydrate, preferably resistant starch, and also discloses various compositions having resistant starch and fats at various levels.

DE-A-19 503 993 discloses compositions comprising n-3 fatty acids and resistant starch to improve glucose tolerance, insulin resistance or hyperlipidemia, in cases of obesity or diabetes mellitus.

WO-A-00 38537 discloses foodstuffs comprising resistant starch with unsaturated fatty acids for preventing colorectal disease, cardiovascular disease and for reducing glycemic index. A specific embodiment discloses a muesli bar having resistant starch and highly unsaturated fatty acids.

### Summary of the invention

The present inventors have now shown that consumption of a diet high in resistant starch and unsaturated fats or lipids results in desirable effects on carbohydrate and fat metabolism. Specifically, it has been shown that consumption of a diet high in resistant starch and unsaturated fats or lipids leads to an increase in fat oxidation.

Accordingly, the present invention provides a composition for consumption in a single meal according to claim 1.

Preferably, the unsaturated fat is present in a proportion of at least 25% by weight of the total fat content.

Preferably the unsaturated fat is selected predominantly from poly-unsaturated fats, with a good balance between omega-3 and omega-6 types, and mono-unsaturated fats.

In a second aspect, the present invention provides a foodstuff comprising a composition of the present invention.

The composition or foodstuff may, for example, be in the form of a tablet, a foodstuff, a component of a prepackaged meal or a component of a calorie-controlled diet.

In a third aspect, the present invention provides a method for producing a composition of the present invention according to claim 16.

### Detailed description of the invention

### Compositions and foodstuffs

The present invention provides compositions comprising high levels of resistant starch and unsaturated fat.

As used in this specification, the term "resistant starch" includes those forms defined as RS1, RS2, RS3 and RS4 as defined in Brown, McNaught and Moloney (1995) Food Australia 47:272-275. Either modified or unmodified resistant starches or mixtures thereof can be used in the present invention. A particular resistant starch can be the product of multiple modifications. Modified resistant starches include breakdown products such as resistant maltodextrins.

One form of resistant starch particularly suitable for the present invention is starch containing resistant starch. Preferably, the starches have an amylose content of at least 40% (w/w), although the amylose content of the starch may vary depending on the plant species from which the starch has been obtained. In a preferred form the starch is from maize having an amylose content of at least 70% (w/w), at least 80% (w/w) or at least 90% (w/w). The starch can also be chemically, physically, or enzymically treated or modified. Chemical modification can be by oxidation, cross-bonding, etherification, esterification, acidification, dextrinisation, or mixtures thereof. Physical modification includes heat-moisture treatment,

Preferably the resistant starches are derived or obtained from maize (corn). It will be appreciated, however, that other sources of resistant starch could be used in the present invention. Examples include cereals like sorghum, wheat, barley, oats. triticale, maize and rice, tubers like potatoes and tapioca, legumes such as peas, and others including starches derived from conventional inbred breeding techniques or from genetically modified plant species.

Starches can also be treated to enhance the resistant starch content by a number of physical or chemical means. Where the starch has been obtained from non-genetically modified plant species, the resulting treated starch can still be termed non-GMO resistant starch. For a number of reasons relating to the regulation of GMO foods and consumer preference, it may be desirable to use only non-GMO starch, whether treated or untreated, in compositions of the invention.

One preferred treatment means is to heat-treat starch in the presence of moisture (heat-moisture treatment) which can be achieved by a number of processes including heating under negative, atmospheric or positive pressure under elevated moisture, or cycling techniques through different temperatures and pressures. Heating can be in the order of 100 to 180°C, preferably around 120 to 150°C and moisture levels of 10 to 80%, preferably 20 to 60%. Repeated autoclaving and rapid cooling can also be used to increase the resistant starch content of starches. It will be appreciated that these processes and conditions can be changed to achieve the desired increase in the level of resistant starch in the starch being treated.

Treatment can also be by solvent extraction to remove fats and/or minerals from the starch.

In WO 94/03049 and WO 94/14342, high amylose starches are disclosed which are resistant starches and include maize starch having an amylose content of 50% (w/w) or more, particularly 80% (w/w) or more, rice starch having an amylose content of 27% (w/w) or more, or a wheat starch having 35% (w/w) or more. Furthermore, particular granular size ranges of starches having an amylose content of 50% or more and enhanced resistant starch content, these starches including maize, barley, and legumes. This invention is not, however, limited to these forms of resistant starch. For example, other forms of resistant starch can be derived from sources such as bananas and tubers such as potatoes and modified forms thereof.

Chemical modifications, such as oxidation, cross-bonding, etherification, esterification, acidification, dextrinisation and the like are well known in this art as being suitable chemical treatments. Similarly, other modifications can be induced physically, enzymically or by other means well known to those skilled in the art.

It may also be useful to modify the degree of enzyme susceptibility of the resistant starch by altering the conformation or structure of the starch. Examples include acid or enzyme thinning and cross bonding using difunctional reagents, heat/moisture treatment and thermal annealing. Modification of the starch may also be carried out by manipulation of the crystalline nature of the starch. Such modification methods are known to the art and starches produced by these methods would be suitable for the present invention.

Preferably, the resistant starch is derived from maize, sorghum, rice, barley, oats, triticale, wheat, legumes, potato, or banana starches. As the amylose content of some starches appears to be related to the resistant starch content, one preferred embodiment is the use of starches having an amylose content of at least 40% (w/w). Resistant starch obtained or derived from maize starch has been found to be particularly suitable for the present invention. In many starch-containing plants, the amylose content does not need to increase to the high levels found in maize in order for them to demonstrate the properties of resistant starch. These properties are likely to be found in wheat [+35% amylose], banana and barley [+30% amylose]; potato, legumes and rice [+27% amylose]. The amount of resistant starch can be demonstratd by the resistance of the starch granule or starch derived material to attack by amylases, irrespective of its amylose content. However, the amylose content can act as an indicator of whether the starch granule will exhibit this property of resistance to amylolysis.

Maize starches having an amylose content of at least 70% (w/w), at least 80% (w/w) or at least 90% (w/w) are preferred as these starches contain high levels of starch granules forming resistant starch.

The term "unsaturated fats" includes unsaturated fatty acid esters in both solid and liquid form. The terms fats, oils, fatty acids and lipids are used interchangeably herein.

Preferably, the fat is a mono-unsaturated fat, a poly-unsaturated fat, an omega-3 fat, or an omega 6 fat. Further vegetable triglycerides relevant to the present invention include those obtained from seeds, beans, fruits, nuts and other plant materials, often obtained by mechanical expelling and/or solvent extraction. Examples which are particularly suitable for use in the present invention are sunflower oil including high and mid oleic varieties, soybean oil, cottonseed oil, canola or rapeseed oil including low linolenic and other modified varieties, flax or linseed oil including high linolenic varieties [Linola], maize or corn oil, olive oil, peanut oil, rice bran oil, palm oil and fractionated palm oils, palm kernel oil, coconut oil and the like.

Triglycerides of animal origin can be used in the present invention and include those obtained from milk and from the processing of cattle, sheep and fish. Preferred examples include n-3 polyunsaturated fatty acids (PUFAs) and n-6 PUFAs, such as fish oils.

Compositions according to the present invention comprise high levels of resistant starch and high levels of unsaturated fat as a proportion of carbohydrate/fat content compared with typical foodstuffs and dietary supplement. Specifically, compositions of the present invention comprise at least 5g of resistant starch and at least 2g of unsaturated fat.

Thus compositions may comprise at least 5, 10, 15 or 20g of resistant starch. Since one of the aims of the present invention is to replace dietary starch of the non-resistant type, such as amylopectin starch, with resistant starch, it is preferred that the resistant starch is present as a significant proportion of the total starch content of the composition. For example the resistant starch may be present in a proportion of at least 10% by weight of the total starch content, preferably at least 15, 20, 25, 30, 35, 40, 50, 60, 70 or 80% by weight of the total starch content. Similarly, it is preferred that resistant starch is present as a significant proportion of the total carbohydrate content of the composition. For example the resistant starch may be present in a proportion of at least 20% by weight of the total carbohydrate content, preferably at least 25, 30, 35, 40, 50, 60 or 75% by weight of the total carbohydrate content. Types of resistant starch that may be included in the compositions are described above.

In relation to the fat/lipid content, compositions typically comprise at least 2g of unsaturated fat or its equivalent. For example compositions may comprise at least 3, 4, 5, 6 or 8g of unsaturated fat [higher for food spreads such as margarine]. It is preferred to include unsaturated fat such that the ratio of resistant starch to unsaturated fat is from about 1:1 to 1:2, although the ratio can be markedly different for an individual food which is used in the context of the diet of an individual.

Since one of the aims of the present invention is to replace saturated fats with unsaturated fats to achieve the desired metabolic effects that have now been shown to occur when both resistant starch and unsaturated fats form a significant element of an individual's diet. it is preferred that the unsaturated fat is present as a significant proportion of the total fat content of the composition. For example the unsaturated fat may be present in a proportion of at least 25% by weight of the total fat content, preferably at least 35, 50, 75 or 80% by weight of the total starch content. In one embodiment, saturated fat is substantially absent from the composition. Types of unsaturated fat that may be included in the compositions are described above.

Compositions may further comprise carbohydrate sources other than resistant starch, saturated fats, flavouring agents, vitamins, minerals, electrolytes, trace elements and other conventional additives. Proteins, particularly proteins resistant to digestion and termed "by-pass proteins or resistant proteins", may also be included to ensure optimal physiological performance or utilisation. If any of these optional ingredients are not present in the composition of the invention, they should normally be supplied as a supplement to the composition of the invention in other elements of the diet, so that an adequate supply of all essential nutritional ingredients is ensured. If the composition of the invention is intended to supply a substantial part of the food intake of a subject, the optional ingredients are preferably present, so that separate intake thereof can be avoided. This is of particular importance for overweight or obese subjects on a weight reduction treatment, by which it is important that all essential nutritional ingredients are supplied in recommended amounts.

Vitamins and minerals may be added to-the composition in accordance with the limits laid down by health authorities. The composition of the invention may comprise all recommended vitamins and minerals. The vitamins will typically include A, B1, B2, B12, folic acid, niacin, panthotenic acid, biotin, C, D, E and K. The minerals will typically include iron, zinc, iodine, cobber, manganese, chromium and selenium. Electrolytes, such as sodium, potassium and chlorides, trace elements and other conventional additives are also added in recommended amounts.

The composition of the invention may take any form which is suitable for human or animal consumption, such as a foodstuff or drink. In one embodiment, the composition is a powdery mixture which is soluble, suspendable, dispersible or emulsifiable in a water-containing liquid such as water, coffee, tea or fruit juice. For such purpose, the composition may be packed in a package intended for covering the total nutrition requirement for a defined period of time, such as three days or a week, whereby the composition will be divided into suitable sub-units of a daily dose, preferably four to five sub-units for women and four to six sub-units for men per daily dosage, which are packed separately before being packed into the package, or the package will be provided with means for aportioning of such sub-units.

In another preferred embodiment, the composition of the invention is a liquid nutritrional preparation in a water-containing liquid, in which the solid ingredients are dissolved, suspended, dispersed or emulsified in an amount of 10 to 40 weight %. When the liquid nutritional preparation is intended for drinking, it will usually comprise a flavouring agent as discussed above.

In a further embodiment, the composition of the invention may be in the form of a solid composition such as a nutritional bar, fruit bar, cookie, or a bakery product such as cake, bread or muffin, or a dairy product such as a low-fat spread or margarine.

Compositions may form part or all of a prepackaged meal, included chilled and frozen ready-made meals.

Compositions may also be formulated as tablets. Since the quantities of resistant starch and unsaturated fat as well as other ingredients such as binders and flavouring agents result in compositions of at least 7 to 8g, the tablets may be relatively large. Consequently, the tablets will typically be formulated such that they can be chewed prior to swallowing. Alternatively, the compositions may be subdivided into a number of tablets.

Thus compositions of the invention may be provided, for example, in the form of consumer meals, drinks, powders, tablets, health foods, nutritional supplements and animal feeds.

The compositions may form all or part of a calorie controlled diet, for example a calorie-controlled diet having an energy content of from 800 to 1200 kcal per day, or more than 1200 kcal per day, such as more than 2000 kcal per day.

### Production of foods high in resistant starch and unsaturated fats

Compositions of the invention and foodstuffs high in resistant starch and unsaturated fats are intended as a partial or full replacement for the carbohydrate and fat intake in the normal diet of individuals. One method of achieving the necessary replacement is to provide nutritional supplements together with a reduction in quantities of food or particular food items in the existing diet of an individual. Another method is to provide normal food items in which the carbohydrate and fat content and composition has been modified to provide a foodstuff with increased levels of resistant starch and unsaturated fat (and lower levels of non-resistant starch carbohydrate and saturated fat).

Accordingly, the present invention provides a method for producing a composition of the invention which method comprises replacing (i) some or all of the carbohydrate content of a composition with resistant starch and (ii) some or all of the saturated fat content of a composition with unsaturated fat.

A method of preparing a foodstuff of the present invention may comprise substituting one or more food components with one or more food components that have a higher resistant starch content and substituting some or all of the saturated fats with unsaturated fats so as to increase the proportion of resistant starch, increase the proportion of unsaturated fats and lower the proportion of saturated fats.

This may be achieved by simple substitution of ingredients during manufacture and/or processing of ingredients, intermediate products or final products to increase the resistant starch and/or unsaturated fat content. A discussion of suitable sources of high resistant starch content ingredients and unsaturated fats is provided above as well as methods for increasing the resistant starch content of starches. Preferably, the resistant starch content is increased to at least 20% by weight of the total carbohydrate content of the foodstuff or composition, more preferably at least 30, 40, 50 or 70% by weight. Preferably the unsaturated fat content is increased to at least 20% by weight of the total fat content of the foodstuff or composition, more preferably at least 30. 40. 50. 60. 70. 80. 90. 95 or 100% by weight.

The above foodstuffs or compositions may be prepared using normal food manufacturing techniques known in the art relevant to any particular foodstuff or composition.

The extent to which the resistant starch and unsaturated fat content can be increased will vary for different food types. By way of example, the amount of resistant starch in currently available white bread is about 1% by weight and the amount of fat (mainly as polyunsaturated fatty acids) is about 2.5% by weight The resistant starch content of bread may be increased to from 6 to 12% by weight. The amount of fat may be increased to at least 6% in normal white bread (and up to at least 30% in speciality foods).

### Modulation carbohydrate/fat metabolism

An object of the present invention is to modify the diet of an individual by increasing their resistant starch intake together with substituting saturated fats with unsaturated effects to achieve the desired metabolic effects. For example, the levels of resistant starch and unsaturated fats, such as polyunsaturated fatty acids, may be increased as compared with the individual's existing dietary intake. In particular, the proportion of resistant starch relevant to total dietary carbohydrate intake and the proportion of unsaturated fats such as polyunsaturated fatty acids relative to total dietary fat intake may be increased.

This manipulation of an individual's diet may be achieved either by a total diet approach or by a single food approach where compositions of the invention rich in resistant starch and unsaturated fats are administered.

Where the total diet approach is used, the increased levels of resistant starch may be provided by one food or food group and the increased levels of unsaturated fats such as polyunsaturated fatty acids may be provided by another food or food group. By way of example, a bakery product, such as bread, which has been specifically formulated to be high in resistant starch may be provided together with a dairy spread formulated to be high in polyunsaturated fatty acids.

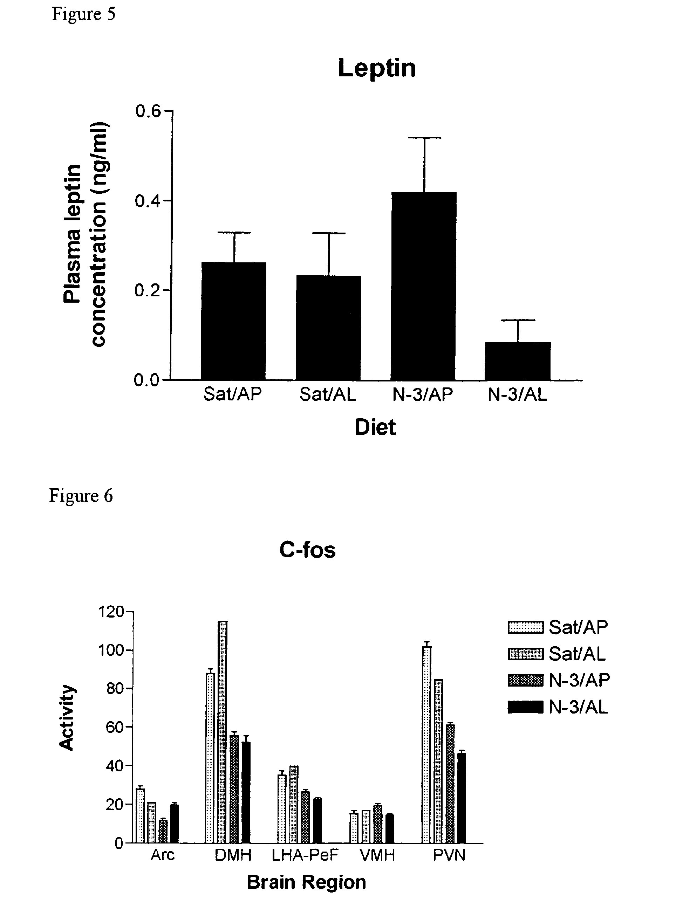

In addition, we have found that transcription of c-fos is modulated significantly in the lateral hypothalamus (LH), ventromedial hypothalamic nucleus (VMH), paraventricular hypothalamic nucleus (PVH), arcuate hypothalamic nucleus (Arc) and dorsomedial hypothalamic nucleus (DMH) (see Figure 6). c-fos transcription is an indicator of neuronal activity (Xin et al., 2000, Brain Research Bulletin, 52: 235-242). Since these regions of the brain are known to play a role in the regulation of energy balance and satiety, the observation that transcriptional activity/neuronal activity in these regions is affected by dietary changes is important.

For example, an individual may be placed on a diet such that in the combined meals, the combined meals contain at least 10 grams of resistant starch or at least 5 grams higher than a comparable meal containing a high quantity of readily digestible starches. It has been found that the consumption of at least 15 grams, preferably at least 20 grams, and more preferably around 30 grams total resistant starch per day with meals provides an improved fat metabolism of fat, namely increased oxidation of dietary fats and/or mobilisation and utilisation of stored fats, in an individual.

Preferably, the high carbohydrate diet rich in resistant starch provides approximately 50% (it may be higher or lower) of the available calories from carbohydrate, with at least 5 g, preferably 10 g, more preferably at least 20 g, even more preferably at least 25 g, and most preferably at least 30 g resistant starch per day. The consumption of at least 5 g of resistant starch, preferably at least 10 g in a single meal will also have a beneficial effect by increasing fat oxidation.

In addition, the diet may also comprise an increased proportion of unsaturated fats. Preferably, the amount of unsaturated fat in the diet is such that at least 50% of the available calories from fat are provided by the unsaturated fat, more preferably at least 70%.

Respiratory Quotient (RQ) is the molar ratio of carbon dioxide (CO₂) produced to oxygen (O₂) consumed and this ratio varies depending on the energy source being utilised by the body. RQ when oxidising carbohydrate as the sole energy source is theoretically 1.00, RQ when oxidising lipids as the sole energy source is theoretically 0.71. Mixed diets will produce RQs which vary between these two theoretical values.

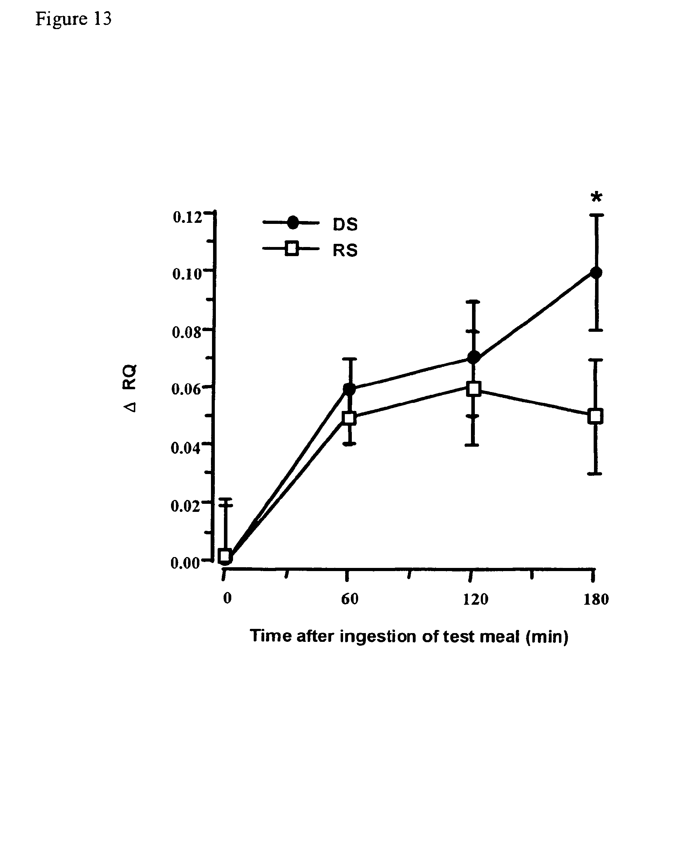

The results shown herein demonstrate that the RQ is lowered in individuals consuming a diet high in resistant starch (see Figure 13). This indicates that the resistant starch is causing a shift in fuel mobilisation that favours fat oxidation over carbohydrate oxidation.

The compositions of the invention may be used to achieve an increase in fat oxidation (which may be evidenced by a reduction in RQ).

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

In order that the present invention may be more clearly understood, preferred forms will be described with reference to the following examples and drawings, which are illustrative only and non-limiting.

### Description of the Figures

**Figure 1****.** Post-prandial plasma glucose concentrations in response to **a)** cooked or **b)** uncooked starches of different amylose concentration. Values for each diet group (n=7) are expressed as means ± s.e.

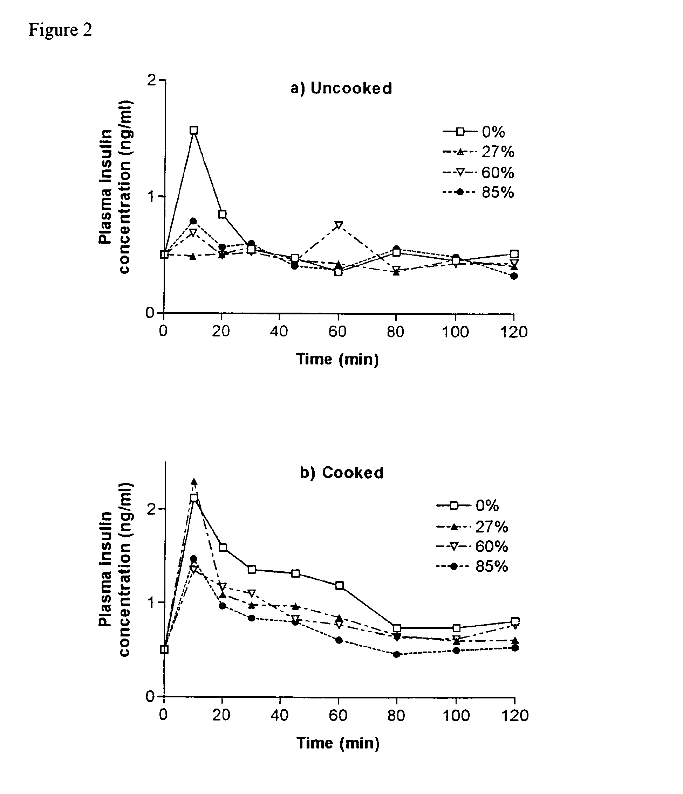

**Figure 2****.** Post-prandial plasma insulin concentrations in response to **a)** cooked or b) uncooked starches of different amylose concentration. Values for each diet group (n=7) are expressed as means± s.e.

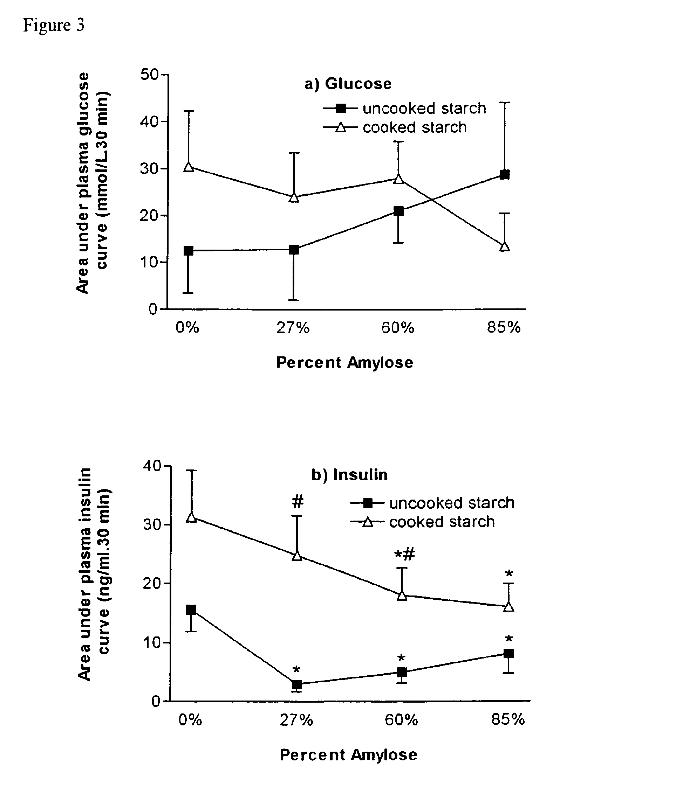

**Figure 3****.** Incremental area under the curve (AUC) for **a)** glucose and **b)** insulin in response to meals of different amylose concentration. (*) represents a significant difference (p=0.05) from the 0% amylose group in the same category (ie cooked or uncooked). (#) represents a significant difference (p=0.05) from the uncooked starch of the same amylose concentration. Values for each diet group (n=7) are expressed as means± s.e.

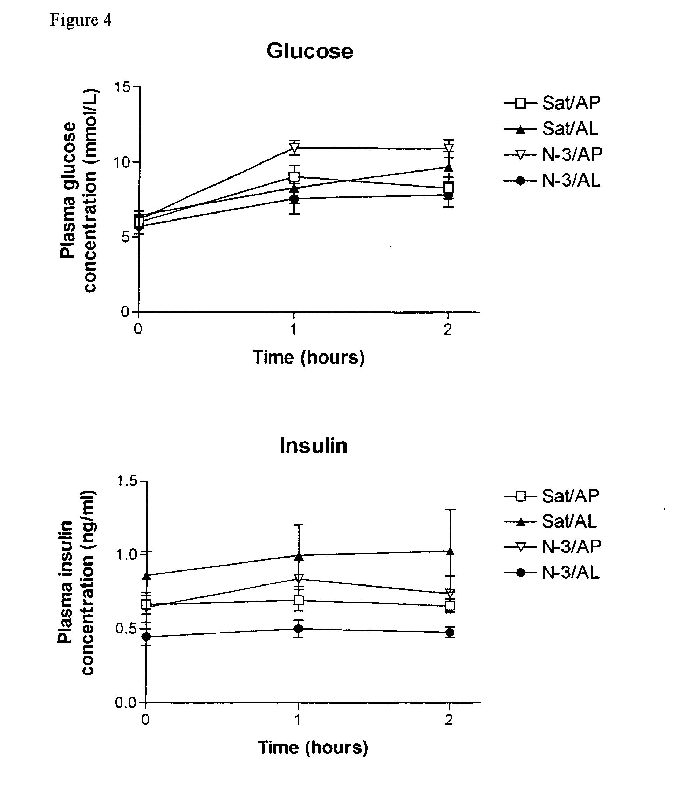

**Figure 4****. a)** Plasma glucose (mmol/L) concentrations of the four dietary groups in response to a 2-hour intravenous glucose challenge (10%). Values for each diet group (n=12) are expressed as means± s.e. The saturated fat/amylopectin diet (Sat/AP) is significantly different from the n-3/amylopectin (N-3/AP) diet (p=0.05), while the n-3/amylose (N-3/AL) diet is significantly different from the n-3/amylopectin diet (N-3/AP) (p=0.001). **b)** Plasma insulin (ng/ml) concentrations of the four dietary groups in response to a 2-hour intravenous glucose challenge (10%): Values for each diet group (n=12) are expressed as means ± s.e. The saturated fat/amylose (Sat/AL) diet is significantly different from the n-3/amylose (N-3/AL) diet (p=0.001), while the n-3/amylopectin (N-3/AP) diet is significantly different from the n-3/amylose diet (N-3/AL) (p=0.05).
**Figure 5****.** Fasting plasma leptin concentrations (ng/ml) in the four dietary groups following a 16-week dietary protocol. Values for each diet group (n=12) are expressed as means± s.e. There were significant differences between the starch groups (p=0.05), but not between fat groups.
**Figure 6****.** Fasting c-fos activation of different hypothalamic regions of the brain in the four dietary groups following a 16-week dietary protocol. Values for each diet group (n=5, *n = 1 for saturated fat*/*amylose group*) are expressed as means± s.e. C-fos values of the various regions of the brain were statistically significant at (p=0,001) for DMH, (p=0.01) for ARC, (p=0.005) for LHA, (p=0.001) for PVN, and (p=0.05) for VMH. [Key: lateral hypothalamus (LH), ventromedial hypothalamic nucleus (VMH), paraventricular hypothalamic nucleus (PVH), arcuate hypothalamic nucleus (Arc) and dorsomedial hypothalamic nucleus (DMH)].
**Figure 7****.** Rate of lipogenesis (microgram atom H/min/g) at 1 hour and 2 hours in brown adipose tissue (BAT) in response to starches of different amylose concentration. Values for each diet group (n=8) are expressed as means ± s.e. Amylose and amylopectin fed rats were sigificantly different at the 2-hour time point (p=0.01).
**Figure 8****.** Rate of lipogenesis (microgram atom H/min/g) at 1 hour and 2 hours in gastrocnemius muscle tissue in response to starches of different amylose concentration. Values for each diet group (n=8) are expressed as means ± s.e.
**Figure 9****.** Rate of lipogenesis (microgram atom H/min/g) at 1 hour and 2 hours in white adipose tissue (WAT) in response to starches of different amylose concentration. Values for each diet group (n=8) are expressed as means ± s.e. Amylose and amylopectin fed rats were sigificantly different at the 1-hour time point (p=0.01).
**Figure 10****.** Rate of lipogenesis (microgram atom H/min/g) at 1 hour and 2 hours in liver tissue in response to starches of different amylose concentration. Values for each diet group (n=8) are expressed as means ± s.e.
**Figure 11****.** Rate of lipogenesis (microgram atom H/min/g) at 1 hour and 2 hours in liver tissue in response to starches of different amylose concentration. Values for each diet group (n=8) are expressed as means ± s.e.
**Figure 12****.** Rate of glycogenesis (microgram atom H/min/g) at 1 hour and 2 hours in gastrocnemius muscle tissue in response to starches of different amylose concentration. Values for each diet group (n=8) are expressed as means ± s.e.
**Figure 13****.** Change in RQ in response to resistance starch in the diet. Two weeks after commencing a DS or RS diet (day 14), subjects returned for a follow-up fasting blood sample and a 3 hour meal test. The test meal consisted of 60 g breakfast cereal, 250 mL Lite White milk, 1 slice of bread (toasted), 1 muffin (toasted), 10g of Canola margarine and 20 g of jam. Results are expressed as mean ± SEM (n = 12 for DS solid circles, n = 11 for RS, open circles). *p < 0.03 for difference from the RS group at the same time point.

### EXAMPLES

The following examples are provided as technical background to the invention and do not form part of the invention.

### Example 1 - Acute Study

Rats were provided with standard rat chow for one week before surgical implantation of canulae. Canulations were then performed one week prior to conducting the acute meal tests. One week post canulation, rats were fasted overnight. The following morning, the rats were presented with one gram carbohydrate /kg body weight and post-prandial blood samples were taken over a 2 hour period. Two hours after eating, the rats were sacrificed and their tissues were harvested for later analysis.

**Table 1. Diet composition of acute meals.**

| **Diet Composition of Acute Meal Test Diet** | |
|---|---|
| **Ingredients** | **grams/kg (diet)** |
| Starch | 514 |
| Sucrose | 85 |
| Methionine | 2 |
| Bran | 50 |
| Gelatine | 19 |
| Sunflower oil (ml/kg) | 25 |
| Canola oil (ml/kg) | 25 |
| Casein | 200 |
| Vitamins | 13 |
| Minerals | 67 |
| **TOTAL** | **1000** |

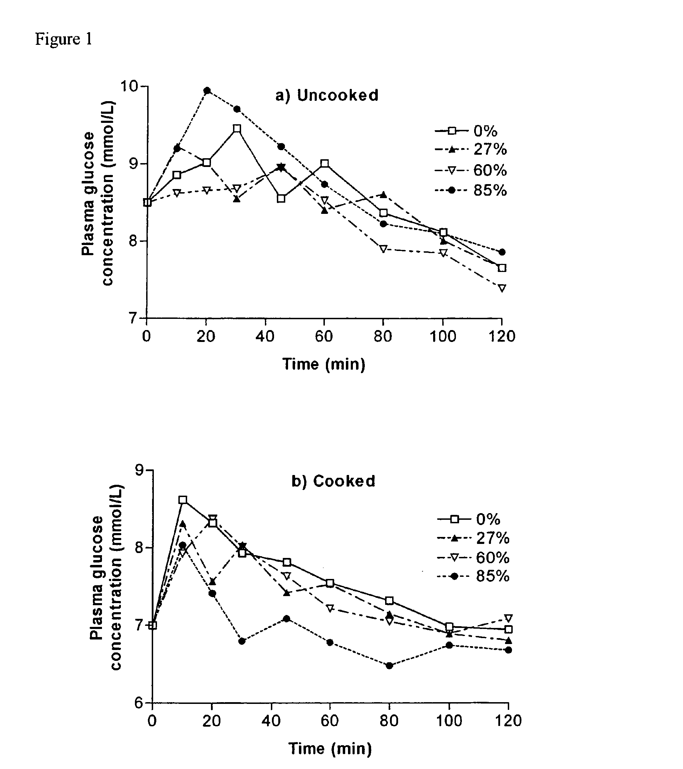

The results are shown in Figures 1- to 3 and Table 2.

### Example 2 - Chronic Study

Offspring of lab bred rats were injected at 2 days of age with Streptozotocin, to induce a non-insulin diabetic condition, or with standard buffer solution. At 8 weeks of age, the rats were fasted overnight and given a glucose tolerance test to determine their diabetic state. Rats were divided into diabetic or non-diabetic groups and fed test diets for 8 weeks. Metabolic rates were obtained on each rat at week 7 of the feeding period. Upon completion of feeding, glucose tolerance tests were repeated and blood samples obtained. Rats were then sacrificed and brains and muscle tissues were harvested for later analysis.
The results are shown as Figures 4 to 6.

**Table 2. Body weights, basal plasma glucose and insulin, and kcal, of diet consumed during meal tests.**

| Diet Group | Weight (g) | Basal Glucose (mM) | Basal Insulin (ng/ml) | kcal Consumed/ kg Body Weight |
|---|---|---|---|---|
| *Uncooked Starch* | | | | |
| 0% | 272.5 ± 11.0 | 8.70 ± 0.22 | 0.50 ± 0.07 | 3.79 + 0.48 |
| 27% | 271.1 ± 6.0 | 8.32 ± 0.64 | 0.51 ± 0.07 | 3.97 ± 0.53 |
| 60% | 266.1 ± 4.3 | 8.48 ± 0.30 | 0.47 ± 0.08 | 4.26 ± 0.20 |
| 85% | 268.8 ± 8.4 | 8.18 ± 0.43 | 0.44 ± 0.05 | 4.18 ± 0.32 |

| *Cooked Starch* | | | | |
|---|---|---|---|---|
| 0% | 305.0 ± 14.0 | 7.41 ± 0.49 | 0.42 ± 0.09 | 4.81 ± 0.52 |
| 27% | 320.8 ± 12.4 | 6.75 ± 0.29 | 0.50 ± 0.14 | 4.39 ± 0.71 |
| 60% | 306.4 ± 16.3 | 7.30 ± 0.22 | 0.53 ± 0.12 | 4.99 ± 0.44 |
| 85% | 319.8 ± 16.9 | 7.38 ± 0.32 | 0.68 ± 0.11 | 5.23 ± 0.70 |

### Example 3 - Absorption Study

Rats were provided with standard rat chow for one week before surgical implantation of canulae. Canulations were then performed one week prior to conducting the acute meal tests. One week post canulation, rats were fasted overnight. The following morning, animals were presented with one gram carbohydrate per kg body weight. After eating, rats were injected with radioactive marker and post-prandial blood samples were taken over a 2 hour period.
Rats were sacrificed at either 1 hour or 2 hours after feeding and their tissues were harvested for later analysis.

**Table 3. Diet Composition, Total Energy, and Percent Energy of the long-term diets.**

| **Ingredients** | **grams/kg (diet)** | **energy (kcal)** | **% energy** |
|---|---|---|---|
| **Sucrose** | 150 | 600 | 12.6 |
| **Protein** | 140 | 560 | 11.8 |
| **Starch** | 450 | 1800 | 37.8 |
| **fat** | 200 | 1800 | 37.8 |
| **fibre** | 50 | | |
| **Vit&min** | 10 | | |
| **TOTAL** | 1000 | 4760 | 100 |

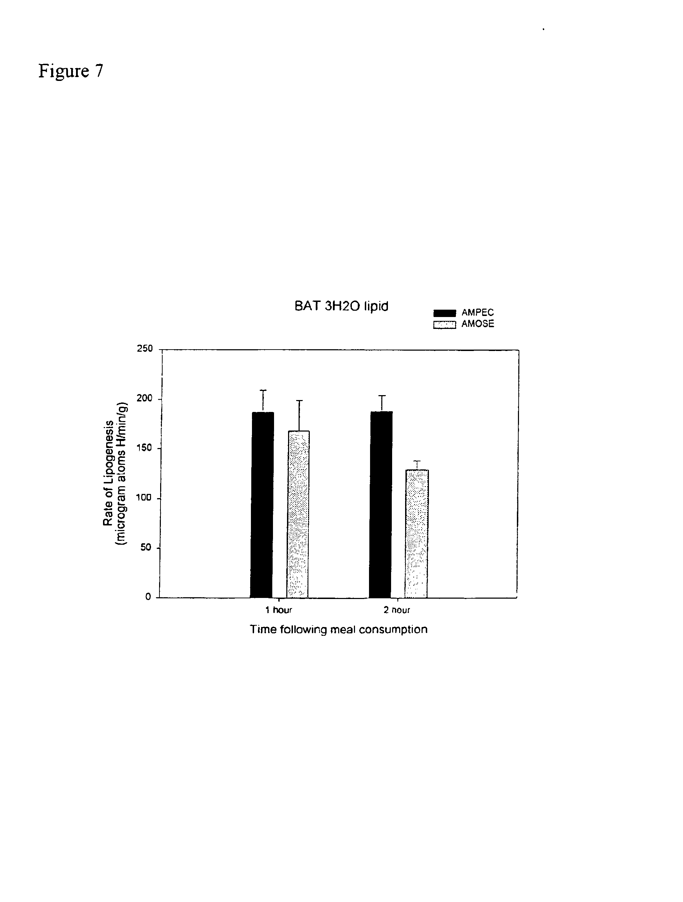

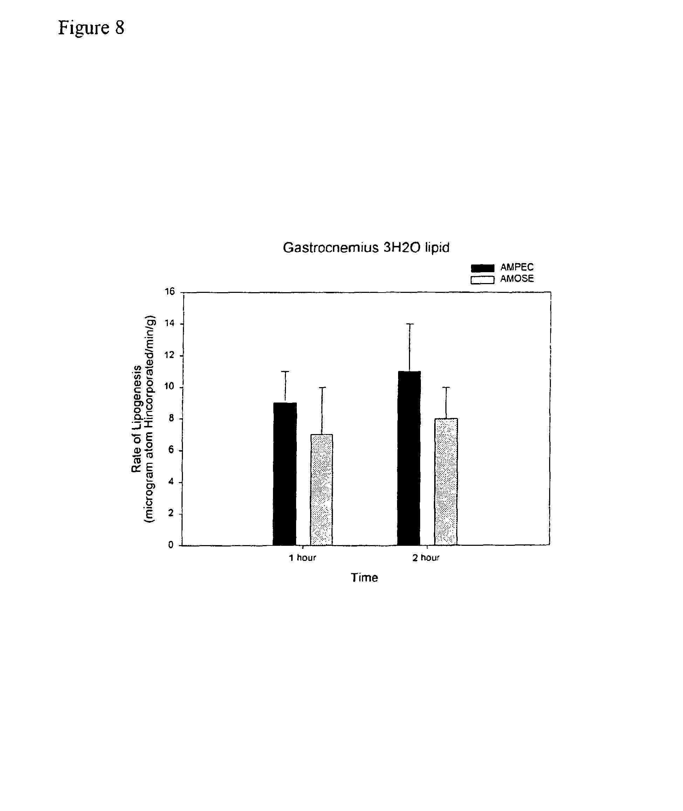

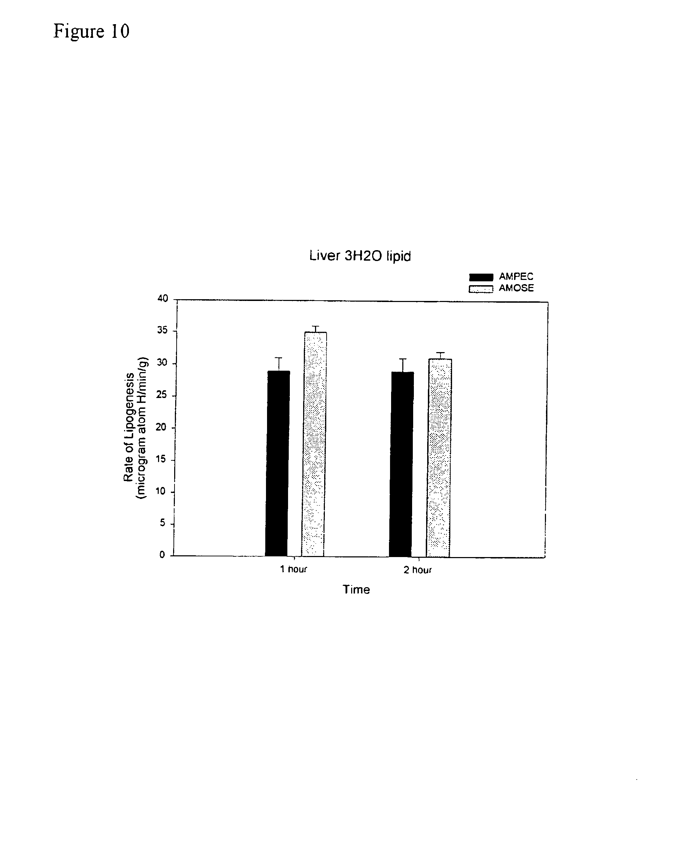

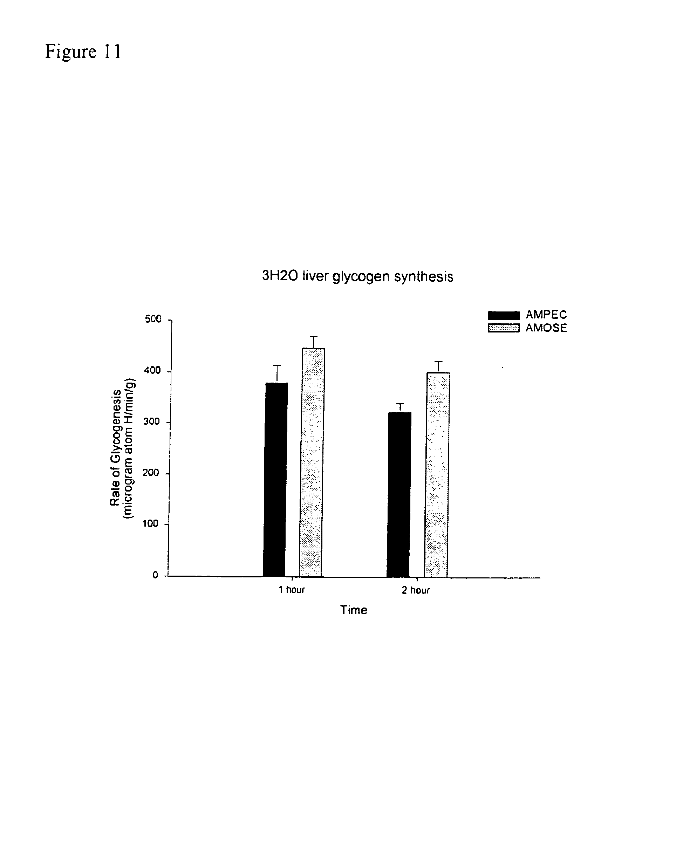

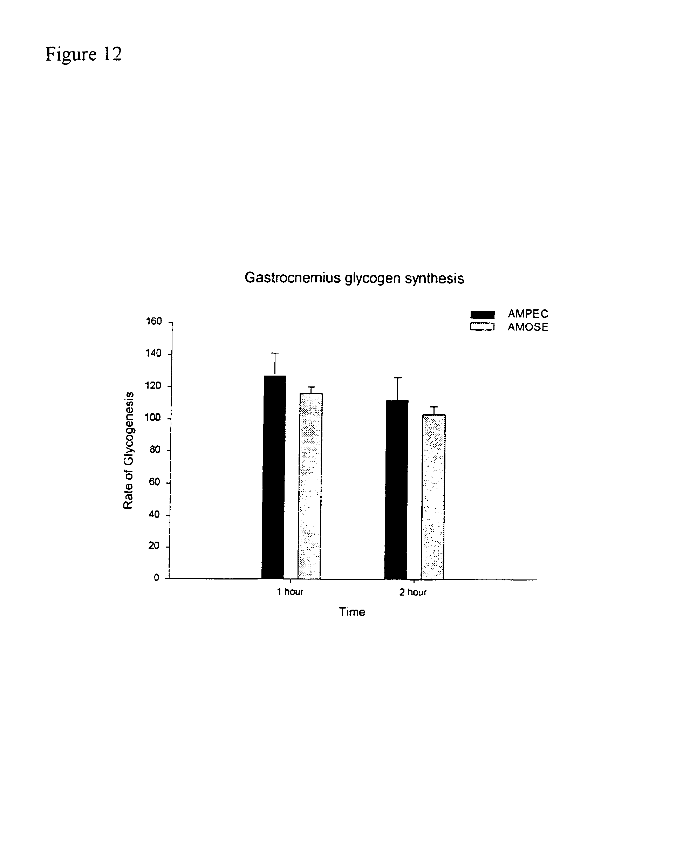

The results are shown as Figures 7 to 12.

### Discussion of results obtained in Examples 1 to 3

### c-fos activity

The effect of diet on neuronal (c-fos) activity is quite interesting, when looking at its impact on total energy balance. The lateral hypothalamus (LHA) is thought to be the feeding center within the parasympathetic system, which is associated with positive energy balance. The ventromedial hypothalamus (VMH), however, is considered the satiety centre of the sympathetic nervous system and represents negative energy balance. Figure 6 illustrates that diets high in unsaturated fats and resistant starch have decreased activation of the hunger centre (LHA) and increased levels of activation of the satiety center, whereas diets high in saturated fat and low in resistant starch have the opposite effect. When taken together (LHA/VMH), these values determine total energy balance.

### Absorption

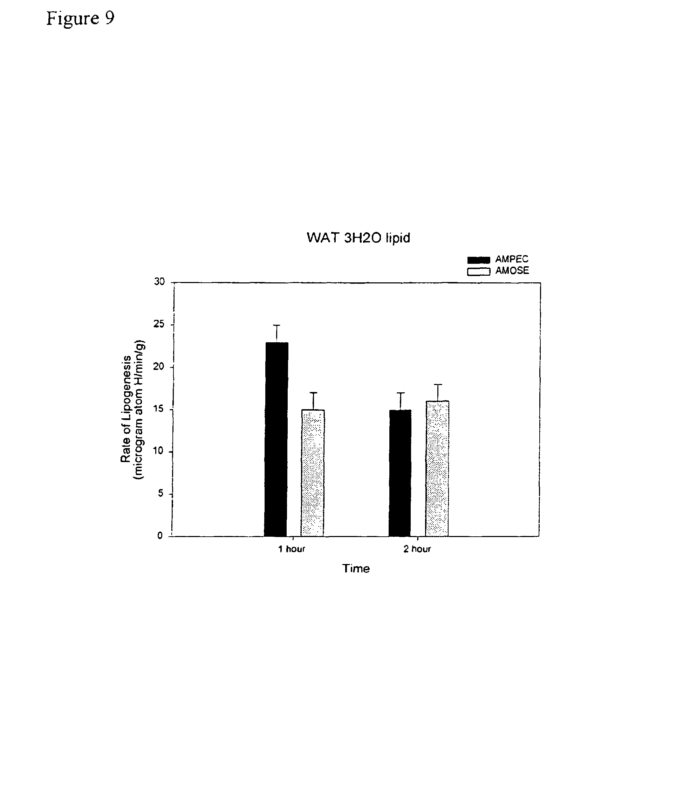

Preliminary results from RQ data suggest a shift in substrate utilisation, from glycogen to fat oxidation, when increasing the proportion or percentage of resistant starch. However, it is unclear if, when and where these changes actually occurred. Figures 7 through 12 illustrate the effect of resistant starch on the glycogen synthesis/utilisation and lipid synthesis/oxidation of various tissues, following ingestion of starches of different concentration. Figures 7 and 9 show significant differences in the rate of lipogenesis within brown adipose and white adipose tissues and a trend toward increased glycogenesis within liver tissue. This confirms that consumption of resistant starch, especially long-term, may in fact shift substrate utilisation from glycogen to fat oxidation.

### Leptin levels

leptin is a protein synthesized in adipose tissue and is thought to inhibit food intake and increase satiety. Leptin receptor is found in the hypothalamus of the brain and may be a key link between the neuronal (c-fos) and hormonal systems and their effect on caloric homeostasis. Although other studies have shown differences in leptin levels between groups fed saturated fat and unsaturated fat, Figure 5 shows significant differences only between groups with different starch concentrations, although there was a trend toward higher leptin levels in the unsaturated fat group. Differences between the starch groups can be explained by the large difference in body and fat weights of the animals, as there was substantial weight loss with chronic resistant starch consumption. This weight loss could be attributed, in part, to the substrate utilisation shift we noted in the absortion study. Also, with the fat loss will be a decrease in leptin production.

### Example 4 - Effect of Resistant Starch Diet on RQ values in Humans

### METHODS

Twenty-four healthy males (19 - 34 years of age) participated in the present study. Approval for this work was granted by the University of Wollongong Human Ethics Committee and full written consent was obtained from all subjects prior to commencement of the trial.

Subjects were randomly divided into two groups. The first group received a traditional starch (TS) diet, low in resistant starch, whereas the second group received a Hi-maize^{™} (HM) diet, high in resistant starch. The TS diet consisted of standard commercially available products whereas the HM diet consisted of commercially available product containing [Hi-maize^{™} (Table 1). For the TS group, mean and SEM values for age, height and weight were 22.3 ± 0.6 years, 180 ± 3.1 cm. and 73.5 ± 3.7 kg, respectively. For the HM group, mean and SEM values for age, height and weight were 23.5 ± 0.6 years, 185 ± 1.8 cm. and 74.1 ± 2.4 kg, respectively.

All subjects were requested to eat at least 60 g breakfast cereal, 4 slices of white bread, and 2 muffins per day plus 3 pasta meals (125 g servings) per week for 14 days. An excess of these foods was provided such that subjects could exceed the intake guidelines if necessary as all participants were exercising regularly (4-8 times per week). All subjects were advised not to eat foods containing a significant amount of resistant starch (eg. legumes, green bananas and bismati rice) during the study in effort to control the 'background' intake of resistant starch (ie. resistant starch from sources other then those provided as part of the study). All foods supplied to subject were donated by Buttercup Bakeries, Uncle Toby's Company Ltd, and New Zealand Starch Products on behalf of Starch Australasia Ltd.

Before commencing the allotted diet (day 0), a fasting venous blood sample (antecubital) was taken from each subject followed by a diet history interview and thorough explanation of the dietary guidelines for the study. Two weeks after commencing the diet (day 14), subjects returned for a follow-up fasting blood sample and a 3 hour meal test. The test meal was either TS or HM, based upon the subject's diet over the two week study period, and consisted of 60g breakfast cereal, 250 ml Lite White milk, 1 slice of bread (toasted), 1 muffin (toasted), 10 g of Canola margarine and 20 g of jam.

Venous blood samples (antecubital) were taken 30, 60, 120 and 180 min post-ingestion of the test meal. Respiratory quotient (RQ) measurements were taken at 0, 60, 120 and 180 min after ingestion of the test meal using a Datex Deltatrac II (Helsinki, Finland). In addition, all blood samples were analysed for serum glucose, serum insulin, plasma cholesterol, plasma total lipid and plasma free fatty acid concentration.

**Table 4. Resistant starch (RS) content of foods consumed (% w/w)**

| | Traditional starch group (TS) | | Hi-maize^{™} group (HM) | |
|---|---|---|---|---|
| | Product | RS content | Product | RS content |
| Cereal | Uncle Toby's MaxNRG | 0.7 | Uncle Toby's Grinners | 3.4 |
| Bread | Buttercup Super Sandwich Maker | 0.8 | Buttercup Wonder White | 2.9 |
| Muffins | Buttercup English Muffins | 0.8 | Wonder White Muffins | 1.6 |
| Pasta | Vetta pasta Spirals | <0.1 | Hansell Pasta Spiral | 1.5 |
| | TOTAL per meal | 2.4 | | 9.4 |

**Table 5. Actual nutrient intake during the dietary intervention as assessed from diet history records and subject's daily food check list. Values indicated as a percentage represent the percentage of total calorie intake.**

| | | |
|---|---|---|
| | Traditional starch group | Hi-maize™ group |
| Energy intake (kj/d) | 13871 ± 3500 | 13258 + 3100 |
| Carbohydrate (%) | 53 ± 1.0 | 57 ± 1.4 |
| Resistant starch (g/d) | 2.4 | 9.4 |
| Protein (%) | 17 ± 0.3 | 16 ± 0.2 |
| Total Fat (%) | 27 ± 0.3 | 24 ± 0.2 |
| Saturated fat (%) | 12 ± 0.1 | 11 ± 0.1 |
| Mono-unsaturated fat (%) | 10 ± 0.1 | 8 ± 0.1 |
| Poly-unsaturated fat (%) | 5 ± 0.1 | 5 ± 0.04 |

### RESULTS

Of the 24 subjects recruited, one subject from the HM group was found to be insulin resistant according to World Health Organisation (WHO) criteria and was eliminated from the study. Total energy intake and macronutrient composition of the diet did not significantly differ between the TS and HM groups (Table 5).

There was no difference in fasting RQ values between the TS and HM groups (data not shown). RQ values ranged between 0 83 and 0.91 and were plotted as ΔRQ which represents the difference between the RQ at each time point and that at O min (Figure 13). The ΔRQ at 60 and 120 min after meal ingestion showed no difference between the TS and HM groups. After 180 min. however, the ΔRQ for the HM group was approximately 50% of that for the TS group.

### Discussion

Two groups of healthy males (age 18-34 years) consumed a high carbohydrate diet containing either traditional starch (TS) products low in resistant starch or Hi-maize^{™} (HM) products high in resistant starch content for two weeks. RQ measurement and blood samples were taken post-meal ingestion to be analysed for glucose, insulin, free fatty acid (FFA), cholesterol and total lipid concentration. At 3 hours the ΔRQ for the HM group (0.04 ± 0.01) was approximately 50% of that for the TS group (0.09 ± 0.02; p < 0.01). This data provides evidence that a diet high in resistant starch causes an acute shift in fuel utilisation that favours fat oxidation over carbohydrate oxidation with the consumption of elevated levels of dietary resistant starch.

In absolute terms, carbohydrate was the primary source of energy at 1, 2 and 3 hours post-meal ingestion as RQ values ranged from 0.90 to 0.92. The decrease in RQ which was observed in the HM group relative to the TS group 3 hours after eating represents an increase in fat oxidation. Although the magnitude of this decrease in RQ (0.05 units) seems small, it accounts for a large difference in fat oxidation. For example, if subjects in the TS group were oxidising 50% fat and 50% carbohydrate, the observed decrease in RQ would mean that subjects in the HM group were oxidising 67% fat and 33% carbohydrate. This substantial difference in fuel utilisation is of particular interest, especially since the difference in the total resistant starch content between the HM and TS diets was relatively low. The meal used for the acute assessment contained approximately four times as much resistant starch as the TS diet (28.2% versus 7.2% (w/w), respectively). A larger increase in the amount of resistant starch may result in an even larger effect on fuel utilisation.

Resistant starch consumption caused an acute increase in fat oxidation. In addition, consumption of a high carbohydrate diet, irrespective of resistant content, lowered fasting plasma FFA concentrations. Taken together, these results indicate that a high carbohydrate diet, rich in resistant starch may be beneficial for those who suffer metabolic diseases in which plasma FFA oversupply is symptomatic such as obesity and non-insulin dependent diabetes mellitus.

## Claims

1. A composition for consumption in a single meal comprising at least 5g of resistant starch and at least 2g of unsaturated fat wherein the resistant starch is present in a proportion of at least 20% by weight of the total carbohydrate content.

2. A composition according to claim 1 comprising at least 10g of resistant starch.

3. A composition according to claim 1 or 2 wherein some or all of the resistant starch is, or is derived from, a high amylose maize starch having an amylose content of 50% or more by weight.

4. A composition according to claims 1 to 3 wherein the unsaturated fat is present in a proportion of at least 25% by weight of the total fat content.

5. A composition according to claim 4 wherein the unsaturated fat is present in a proportion of at least 50% by weight of the total fat content.

6. A composition according to claim 5 from which saturated fats are substantially absent.

7. A composition according to any one of claims 1 to 6 wherein the unsaturated fat is selected from one or more of a mono-unsaturated fat, a poly-unsaturated fat, an omega-3 fat, and an omega 6 fat.

8. A composition according to any one of claims 1 to 7 which further comprises at least one further ingredient selected from the group consisting of a flavouring agent, a vitamin source, a mineral source, an electrolyte, and a trace element.

9. A composition according to any one of claims 1 to 8 in the form of a low calorie diet having an energy content of from 3349.44 to 5024.16 kJ (800 to 1200 (kcal)) per day.

10. A composition according to any one of claims 1 to 8 in the form of a diet having an energy content of more than 5024.16 kJ (1200 (kcal)) per day.

11. A composition according to any one of claims 1 to 8 in the form of a diet having an energy content of more than 8373.6 kJ (2000 (kcal)) per day.

12. A composition according to any one of claims 1 to 11 in the form of a powdery mixture, said powdery mixture being soluble, suspendable, dispersible or emulsifiable in a water-containing liquid.

13. A composition according to any one of claims 1 to 11 in the form of granules.

14. A foodstuff comprising a composition according to any one of claims 1 to 13.

15. A foodstuff according to claim 14 which is provided as a prepackaged meal comprising at least one meal component which comprises said composition.

16. A method for producing a composition according to any one of claims 1 to 13 which method comprises replacing (i) some or all of the carbohydrate content of the composition with resistant starch and (ii) some or all of the saturated fat content of the composition with unsaturated fat.

17. A composition according to anyone of claims 1 to 13 for increasing fat oxidation.

## Patentansprüche

1. Zusammensetzung zum Verzehr in einer einzelnen Mahlzeit, umfassend wenigstens 5 g beständige Stärke und wenigstens 2 g ungesättigtes Fett, wobei die beständige Stärke in einem Anteil von wenigstens 20 Gew.-% des gesamten Kohlenhydratgehalts vorliegt.

2. Zusammensetzung gemäß Anspruch 1, umfassend wenigstens 10 g beständige Stärke.

3. Zusammensetzung gemäß Anspruch 1 oder 2, wobei ein Teil der beständigen Stärke oder die gesamte beständige Stärke eine Hochamylose-Maisstärke mit einem Amylosegehalt von 50 Gew.-% oder mehr ist oder davon abgeleitet ist.

4. Zusammensetzung gemäß Ansprüchen 1 bis 3, wobei das ungesättigte Fett in einem Anteil von wenigstens 25 Gew.-% des gesamten Fettgehalts vorliegt.

5. Zusammensetzung gemäß Anspruch 4, wobei das ungesättigte Fett in einem Anteil von wenigstens 50 Gew.-% des gesamten Fettgehalts vorliegt.

6. Zusammensetzung gemäß Anspruch 5, bei der im Wesentlichen keine gesättigten Fette vorliegen.

7. Zusammensetzung gemäß einem der Ansprüche 1 bis 6, wobei das ungesättigte Fett aus einem oder mehreren von einem einfach ungesättigten Fett, einem mehrfach ungesättigten Fett, einem omega-3-Fett und einem omega-6-Fett ausgewählt ist.

8. Zusammensetzung gemäß einem der Ansprüche 1 bis 7, die ferner wenigstens einen weiteren Bestandteil umfasst, der ausgewählt ist aus der Gruppe bestehend aus einem geschmacksgebenden Mittel, einer Vitaminquelle, einer Mineralstoffquelle, einem Elektrolyten und einem Spurenelement.

9. Zusammensetzung gemäß einem der Ansprüche 1 bis 8 in der Form einer Niederkalorie-Diätkost mit einem Energiegehalt von 3349,44 bis 5024,16 kJ (800 bis 1200 (kcal)) pro Tag.

10. Zusammensetzung gemäß einem der Ansprüche 1 bis 8 in der Form einer Diätkost mit einem Energiegehalt von mehr als 5024,16 kJ (1200 (kcal)) pro Tag.

11. Zusammensetzung gemäß einem der Ansprüche 1 bis 8 in der Form einer Diätkost mit einem Energiegehalt von mehr als 8373,6 kJ (2000 (kcal)) pro Tag.

12. Zusammensetzung gemäß einem der Ansprüche 1 bis 11 in der Form eines pulverförmigen Gemischs, wobei das pulverförmige Gemisch in einer wasserhaltigen Flüssigkeit löslich, suspendierbar, dispergierbar oder emulgierbar ist.

13. Zusammensetzung gemäß einem der Ansprüche 1 bis 11 in der Form von Granulatkörnern.

14. Nahrungsmittel, umfassend eine Zusammensetzung gemäß einem der Ansprüche 1 bis 13.

15. Nahrungsmittel gemäß Anspruch 14, das als abgepackte Mahlzeit bereitgestellt ist, umfassend wenigstens eine Mahlzeitkomponente, welche die Zusammensetzung umfasst.

16. Verfahren zum Herstellen einer Zusammensetzung gemäß einem der Ansprüche 1 bis 13, umfassend das Ersetzen (i) eines Teils oder des gesamten Kohlenhydratgehalts der Zusammensetzung durch beständige Stärke und (ii) eines Teils oder des gesamten Gehaltes an gesättigtem Fett der Zusammensetzung durch ungesättigtes Fett.

17. Zusammensetzung gemäß einem der Ansprüche 1 bis 13 zum Erhöhen der Fettoxidation.

## Revendications

1. Composition à consommer en un seul repas comprenant au moins 5 g d'amidon résistant et au moins 2 g de graisse insaturée, l'amidon résistant étant présent en une proportion au moins égale à 20 % en poids de la teneur totale en glucides.

2. Composition selon la revendication 1, comprenant au moins 10 g d'amidon résistant.

3. Composition selon la revendication 1 ou 2, dans laquelle une partie ou la totalité de l'amidon résistant est un amidon de maïs riche en amylose ou est dérivée d'un amidon de maïs riche en amylose ayant une teneur en amylose supérieure ou égale à 50 % en poids.

4. Composition selon les revendications 1 à 3, dans laquelle la graisse insaturée est présente en une proportion au moins égale à 25 % en poids de la teneur totale en graisses.

5. Composition selon la revendication 4, dans laquelle la graisse insaturée est présente en une proportion au moins égale à 50 % en poids de la teneur totale en graisses.

6. Composition selon la revendication 5, sensiblement exempte de graisses insaturées.

7. Composition selon l'une quelconque des revendications 1 à 6, dans laquelle la graisse insaturée est choisie parmi un ou plusieurs des éléments suivants : une graisse monoinsaturée, une graisse polyinsaturée, une graisse oméga 3 et une graisse oméga 6.

8. Composition selon l'une quelconque des revendications 1 à 7, qui comprend en outre au moins un ingrédient supplémentaire choisi dans le groupe constitué d'un aromatisant, d'une source de vitamines, d'une source de minéraux, d'un électrolyte et d'un oligoélément.

9. Composition selon l'une quelconque des revendications 1 à 8, sous forme d'un régime alimentaire hypocalorique ayant une teneur énergétique allant de 3349,44 à 5024,16 kJ (800 à 1200 (kcal)) par jour.

10. Composition selon l'une quelconque des revendications 1 à 8, sous forme d'un régime alimentaire ayant une teneur énergétique supérieure à 5024,16 kJ (1200 (kcal)) par jour.

11. Composition selon l'une quelconque des revendications 1 à 8, sous forme d'un régime alimentaire ayant une teneur énergétique supérieure à 8373,6 kJ (2000 (kcal)) par jour.

12. Composition selon l'une quelconque des revendications 1 à 11, sous forme d'un mélange pulvérulent, ledit mélange pulvérulent pouvant être dissous, mis en suspension, dispersé ou émulsionné dans un liquide contenant de l'eau.

13. Composition selon l'une quelconque des revendications 1 à 11, sous forme de granules.

14. Produit alimentaire comprenant une composition selon l'une quelconque des revendications 1 à 13.

15. Produit alimentaire selon la revendication 14, qui est fourni sous forme d'un repas préemballé comprenant au moins un composant de repas qui comprend ladite composition.

16. Procédé de production d'une composition selon l'une quelconque des revendications 1 à 13, ledit procédé consistant à remplacer (i) une partie ou la totalité de la teneur en glucides de la composition par de l'amidon résistant et (ii) une partie ou la totalité de la teneur en graisses saturées de la composition par de la graisse insaturée.

17. Composition selon l'une quelconque des revendications 1 à 13, destinée à accroître l'oxydation des graisses.
